Europäisches Patentamt

(19) European Patent Office

Office européen des brevets

(11) Publication number: **0 089 331**
**B1**

(12) **EUROPEAN PATENT SPECIFICATION**

(45) Date of publication of patent specification: **23.01.85**

(21) Application number: **83870022.7**

(22) Date of filing: **15.03.83**

(51) Int. Cl.⁴: **C 07 C 87/60,** C 07 C 85/24 //
**C07C147/14**

(54) Manufacture of ortho-methyl anilines from ortho-amino benzyl sulfoxides.

(30) Priority: **17.03.82 US 358772**

(43) Date of publication of application:
**21.09.83 Bulletin 83/38**

(45) Publication of the grant of the patent:
**23.01.85 Bulletin 85/04**

(84) Designated Contracting States:
**AT BE CH DE FR IT LI LU NL SE**

(56) References cited:
**US-A-3 985 756**

(73) Proprietor: **MONSANTO COMPANY**
**Patent Department 800 North Lindbergh**
**Boulevard**
**St. Louis, Missouri 63166 (US)**

(72) Inventor: **Chupp, John Paul**
**29 Lemp Drive**
**Kirkwood Missouri 63122 (US)**
Inventor: **Balthazor, Terry Mack**
**7208 Chamberlain Avenue**
**University City Missouri 63130 (US)**

(74) Representative: **Lunt, John Cooper et al**
**Monsanto Europe S.A. Patent Department**
**Avenue de Tervuren 270-272 Letter Box No 1**
**B-1150 Brussels (BE)**

# Description

## Background of the invention

### 1. Field of the invention

This invention relates to a process for the preparation of ortho-methyl anilines from ortho-amino benzyl sulfoxides.

### 2. Description of the prior art

Sulfoxides of the general formula RSOR are known to undergo rearrangements under acid conditions to produce alpha-substituted sulfides, the overall result being reduction of the sulfoxide group and oxidation of the adjacent carbon atom. Reactions of this type are generally known as the Pummerer reaction. The products of such Pummerer reactions when acid halides are employed normally include alpha-substituted halomethyl sulfides, or via hydrolysis, the corresponding aldehydes. Russell & Mikol, Mech. Mol. Migr., 1, 157—207 (1968).

In the preparation of certain aniline derivatives useful as herbicides, various methods have been developed, for example, for the reduction of ortho-methylthiomethyl anilines or ortho-methylsulfinylmethyl anilines to ortho-methyl anilines. One such reaction involves use of the prior art direct hydrogenation of the sulfide in the presence of a catalyst such as Raney nickel. Use of Raney nickel for desulfurization, however, requires substantial amounts of catalyst, special equipment and special handling.

While use of an ortho-amino benzyl sulfoxide as a starting material to produce anilines can be attractive, particularly when the aniline contains other ring substituents, the presence of sulfur in the starting material is detrimental if hydrogenation employing metal hydrogenation catalyst, such as noble metal catalysts, is contemplated.

It is an object of this invention to provide a process for the conversion of ortho-amino benzyl sulfoxides to ortho-methyl anilines in high yields.

It is a further object of this invention to provide a process for the conversion of ortho-amino benzyl sulfoxides to ortho-methyl anilines that includes an effective separation of sulfur by-products.

It is a still further object of this invention to provide a process for the conversion of ortho-amino benzyl sulfoxides to ortho-methyl anilines that does not include any potentially hazardous process step.

It is another object of this invention to provide a process for the conversion of ortho-amino benzyl sulfoxides to ortho-methyl anilines that employs only readily available reagents.

It is also another object of this invention to provide a useful process for the conversion of anilines derived from sulfilimine rearrangement to ortho-methyl anilines.

## Summary of the invention

This invention provides a process for preparing an ortho-methyl aniline which comprises:

a) reacting an ortho-amino benzyl sulfoxide with a nonoxidizing acid halide in an inert solvent to produce a solution of the corresponding ortho-amino benzyl halide and sulfur by-products;

b) adding to the solution containing said ortho-amino benzyl halide and sulfur by-products a hydrogen halide to form and precipitate an anilinium halide;

c) separating said precipitated anilinium halide from said mixture while retaining the sulfur compounds in solution;

d) neutralizing said anilinium salt and forming a solution of ortho-amino benzyl halide in an inert solvent; and

e) catalytically hydrodehalogenating said ortho-amino benzyl halide to form an ortho-methyl aniline.

This invention provides a facile means of converting ortho-amino benzyl sulfoxides to ortho-methyl anilines in high yields employing readily available reactants. The anilinium salts that are found in the process of this invention can be separated from the reaction medium and from the sulfur by-products which result from the treatment of the sulfoxides with the acid halide. Failure to provide for an effective removal of sulfur compounds from the system, inter alia, results in decreased efficiency in subsequent hydrogenation because hydrogenation catalysts are susceptible to poisoning by sulfur.

Ortho-methyl anilines have a variety of known uses including use in the manufacture of herbicides and the like.

## Detailed description of the invention

A wide variety of ortho-amino benzyl sulfoxides may be employed in the practice of this invention. Importantly, ortho-amino benzyl sulfoxides have been shown to convert readily to ortho-amino benzyl halides with an acid halide and such solution can then be treated in accordance with the further aspect of this invention to provide a substantially sulfur-free stable ammonium salt. A variety of other substituents can be present on the sulfoxide starting material without interfering with the reactions of this invention.

The sulfoxide starting materials may have, if desired, any of a variety of ring substituents in addition to the benzylic sulfoxide substituent. Such secondary nuclear substituents can include, for example, one or more substituents which are alkyl, haloalkyl, alkoxy, polyalkoxy or alkoxyalkyl, alkenyl, alkenyloxy, alkynyl or alkynyloxy, aryl, aryloxy, aralkyl or aralkyloxy, amino, $NO_2$, CN, halogen, and saturated or unsaturated heterocyclic radical having up to 6 ring atoms containing O, S and/or N. Preferred secondary nuclear substituents include haloalkyl, such as $CF_3$; alkyl, such as methyl or ethyl; alkoxy, such as methoxy or ethoxy; halogen, such as Cl or Br, carboalkoxy such as carbomethoxy; and CN. The amino group is an electron donating group that stabilizes the benzyl carbonium ion and results in the formation of the benzyl halide when the benzyl sulfoxide is contacted with an acid halide. Care should, of course, be taken in the selection of secondary electron-withdrawing groups to avoid off-setting the activating effect of the amino group.

The aniline N can have one of a variety of substituents, if desired. Such substituents include, *inter alia*, alkyl or aryl substituent.

The term "benzylic sulfoxide substituent" is employed herein to refer to a $-CH_2SOR$ substituent. The R substituent can be any of a variety of organic substituents such as alkyl, aryl or the like as described above. Since this R group is cleaved from the final product, simple and inexpensive alkyl substituents such as $CH_3$ are preferred.

As used herein, the term "alkyl" refers to both straight chain and branched chain alkyl radicals, preferred are alkyls containing 1 to 6 carbon atoms such as methyl, ethyl, n-propyl, isopropyl, n-butyl, isobutyl, sec-butyl, tert-butyl, n-pentyl, isopentyl, n-hexyl, sec-hexyl and the like; "aryl" refers to substituted and unsubstituted aromatic radicals such as phenyl, tolyl, xylyl and the like; "alkoxy" refers to both straight chain and branched chain alkoxy radicals containing alkyl, alkenyl or alkynyl groups as defined herein; "carboalkoxy" refers to radicals of the formula $COOR_c$ where $R_c$ is an alkyl as defined above; and "amino" as applied to secondary ring substituents refers to radicals of the formula NRR' where R and R' can be hydrogen, an alkyl, aryl or any other substituent as defined above. "Alkenyl" refers to straight chain and branched chain alkenyl groups, of the type $-C_nH_{2n-1}$, preferred are those alkenyl groups containing 3 to 5 carbon atoms; "alkynyl" radicals are of the type $-C_nH_{2n-3}$ and includes both straight chain and branched chain groups. Preferred alkynyl groups contain 3 to 5 carbon atoms. "Alkoxyalkyl" refers to an alkyl group, substituted on the terminal carbon by an alkoxy group.

The term "haloalkyl" refers to alkyl groups substituted by one or more halogen atoms, e.g., chloromethyl, bromomethyl, dichloroethyl, trichloromethyl, trifluoromethyl, pentafluoroethyl, iodomethyl and the like.

Of special interest are those starting materials comprising nuclear substituted ortho-amino benzyl sulfoxides. Of particular interest are 3-substituted-2-amino benzyl sulfoxides (to produce 2—6 di-substituted anilines). 3-trifluoromethyl-2-amino benzyl sulfoxides are especially preferred as starting materials for this invention.

The sulfoxide starting material for the processes of the present invention may be formed, *inter alia*, by known sulfilimine rearrangement from the corresponding aniline. In a typical reaction, an aniline is reacted with dimethyl sulfide in the presence of a base and an oxidizing agent, such as N-chlorosuccinimide, to provide an aromatic sulfilimine containing an $-N=S-C(CH_3)_2$ group. The free sulfilimine may be heated or subjected to catalysis to cause sulfilimine rearrangement to provide an ortho-methylthiomethyl aniline (an ortho-amino benzyl sulfide) which in turn can be oxidized to yield an ortho-methylsulfinylmethyl aniline (i.e., an aniline containing an ortho-$CH_2SOCH_3$ substituent — an ortho-amino benzyl sulfoxide). Such reactions are known and described, *inter alia*, in Gassman, Tetrahedron Letters 497 (1972), Gassman, Tetrahedron Letters, *24*, 2055—2058 (1977), Vilsmeier, Tetrahedron Letters 624 (1972), Jackson U.S. Patents 3,966,371 and 4,006,183, and Claus, Mh Chem. Bd. 102, pp. 1571—1582 (1971). In a variation of the sulfilimine reaction, when a base such as sodium hydroxide is used, the neutralization can be accompanied by a conversion of by-product succinimide to an aqueous solution of sodium succinimide which can be regenerated to a chlorosuccinimide. The sulfilimine rearranges upon heating or catalysis to provide ortho-methylthiomethyl anilines which can in turn be oxidized, for example, with hydrogen peroxide to yield the starting ortho-methylsulfinylmethyl anilines. As described more fully later, ortho-amino benzyl sulfides may be converted to the ortho-amino benzyl sulfoxides and then to the ortho-benzyl halide *in situ* in the practice of this invention.

There are two preferred pathways to convert ortho-amino benzyl sulfides to ortho-amino benzyl sulfoxides. An ortho-amino benzyl sulfide may be oxidized as with hydrogen peroxide to form the corresponding sulfoxide. Alternatively, the sulfide may be contacted with a halogenating agent such as chlorine or sulfuryl chloride under anhydrous conditions to form a cyclic sulfilimine which thereafter may be contacted with water to form the ortho-amino benzyl sulfoxide starting material for the practice of this invention.

If the sulfoxide starting material is prepared by contacting a sulfide with a halogenating agent under anhydrous conditions (i.e., in an anhydrous inert organic solvent) followed by hydrolysis, the sulfoxide can be converted to the ortho-amino benzyl halide *in situ* if sufficient water (e.g., at least about a stoichiometric amount) is added. In this embodiment, the hydrogen halide generated in the formation and hydrolysis of the cyclic sulfilimine can supply the necessary acid halide to convert the sulfoxide to an ortho-amino benzyl halide in a single reaction vessel.

The ortho-amino benzyl sulfoxide starting material is contacted with a nonoxidizing acid halide in an inert solvent to produce an ortho-amino benzyl halide. The term "acid halide" as used herein, refers to agents which are capable of liberating a halide ion (i.e., chloro, fluoro, bromo or iodo) *in situ*. An acid halide for the purposes of this invention can be chosen from a wide variety of acid derivatives such as those derived from sulfonic acids, phosphoric acids, phosphonic acids, and carboxylic acids having an organic moiety that may be alkyl, haloalkyl, phenyl, benzyl or substituted derivatives thereof. Included in this class of materials are acyl halides such as acetyl chloride, and haloacetyl halides such as chloroacetyl chloride. Acid halides also, of course, include hydrogen halides and, indeed, hydrogen chloride is a preferred acid halide. In general, acid halides used in the normal Pummerer reaction can be employed in the abnormal Pummerer reaction described herein. The reaction of the ortho-amino benzyl sulfoxide and the acid halide may be carried out in any of a variety of inert solvents, such as a hydrocarbon, chlorohydrocarbon, ether solvents or the like. Representative solvents include carbon tetrachloride, toluene, xylene, chlorobenzene, chloroform, methylene chloride, ethylene dichloride, trichloroethylene. A preferred solvent is ethylene dichloride.

The ratios of reactants in the above-described process are dictated primarily by economic considerations and avoidance of unwanted by-products. Hence, large excesses or deficiencies of any expensive component relative to another component should be avoided and essentially stoichiometric ratios are often preferred. Concentrations of reactants employed can affect product yield. In general, reactant concentrations of from about 0.1 $M$ up to about 1.5 $M$ can be employed, and yields tend to be optimum at or near approximately 0.5 $M$ concentration.

The process may be carried out at any convenient temperature ranging from 0°C to ambient or higher. Thus, reaction temperatures of from about 0° to about 200°C can be broadly employed. In practice, however, it is preferred to employ temperatures in the range of from about 40° to about 120°C, with temperatures of from about 50° to about 85°C being the most preferred for the reaction. The process may be carried out under any convenient pressure either above or below atmospheric. For practical considerations, however, atmospheric conditions are preferred. The reaction proceeds rapidly. The choice of temperatures, pressures, equipment and the like to suit any particular set of reactants is within the skill of the art. The process also may be carried out, of course, either batchwise or continuously. In the preferred embodiment, the sulfoxide reactant is dissolved in the inert solvent with mixing and gaseous acid halide reagent is admitted, e.g., bubbled through the mixture. In most cases, it is desirable to remove water formed in the reaction by distilling the mixture until the reaction product is essentially anhydrous. Distillation also serves to insure removal of unreacted acid halide.

The conversion of the ortho-amino benzyl sulfoxide to ortho-amino benzyl halide as described above may be characterized as an abnormal Pummerer reaction. This reaction proceeds to the ortho-amino benzyl halide rather than to an aldehyde as in the case with a normal Pummerer reaction.

The inventors of the present invention discovered the abnormal Pummerer reaction and its applicability, *inter alia*, in conversion of ortho-amino benzyl sulfoxides to amino benzyl halides. That process as well as the conversion of benzyl sulfides to benzyl sulfoxides is described in greater detail in the co-pending EP—A—0089330 entitled "Preparation of Substituted Benzylic Halides" filed on even date herewith.

The resulting ortho-amino benzyl halide is stable while dissolved in the solution. However, since the ortho-amino benzyl halides described herein will polymerize if the solvent is removed, these materials are recovered according to this invention as the anilinium halide salts. Such recovery can be effected by treating the benzyl chloride solution with HCl and stirring. Temperatures used for precipitation will, of course, vary with the individual compounds and solvent systems. Temperatures can range from about 150°C or higher to as low as about −10°C or lower. Since some of the anilinium halide salts are formed only at low temperatures, it is preferred to cool the ortho-amino benzyl halide solution to below ambient and often to below about 15°C during the hydrogen halide treatment. The precipitation and filtration of the anilinium salt results in salt yields of up to 95% or more and provides a product which is substantially free from sulfur-containing abnormal Pummerer reaction by-products such as $CH_3SO_2SCH_3$ and $CH_3SH$. This recovery step is particularly important when, as described below, the anilinium salt is further treated, via the hydrodehalogenation route, to produce ortho-methyl anilines. Hydrogenation catalysts typically employed in such hydrodehalogenations are often poisoned by even small quantites of sulfur compounds.

The solvent that is selected for this process step should have limited capability to dissolve the anilinium salt but have the ability to retain the sulfur compounds in solution at operating temperatures. The choice of a solvent from among the many available and the determination of appropriate amounts of solvent to be employed for any given combination of reactants can readily be determined employing simple solubility tests.

The production of useful substituted ortho-methyl anilines from the separated anilinium salts will now be described. This aspect of the process includes neutralization of the salt followed by hydrodehalogenation to provide ortho-methyl anilines in good yields.

The anilinium salt is neutralized and the resulting ortho-amino benzyl halide is dissolved in an inert solvent. While any inert solvent can be employed, the solvent is preferably a polar solvent such as an ester or ether that is non-reactive with the ortho-amino benzyl halide, or the ortho-methyl aniline product, and which is conducive to the subsequent hydrogenation step. Esters constitute a preferred class of solvents. Nucleophilic solvents, such as alcohols, are not preferred since ortho-amino benzyl halides are susceptible to nucleophilic displacement.

The base employed to neutralize the anilinium salt may be either organic (e.g., triethylamine) or inorganic (e.g., $Na_2CO_3$). A wide variety of bases can be employed since the function of the base is merely to neutralize the hydrogen chloride and permit the formation of an ortho-amino benzyl halide.

Neutralization can be accomplished in a number of ways. The anilinium salt can be contacted with an aqueous solution of the base to neutralize and extract the halide and thereafter the benzyl halide can be dissolved in an inert solvent. Alternatively, the anilinium salt can be contacted with a mixture of an aqueous base and a water immersible organic solvent to neutralize the anilinium salt and to dissolve the benzyl halide in the organic solvent. Phase separation then separates the neutralized halogen salts from the benzyl halide solution. In still another alternative, an organic base may be employed which forms halides that are soluble in the organic solvent. It is generally desirable to accomplish neutralization at ambient temperatures or below to avoid unwanted by-products. In the preferred neutralization procedure, the anilinium salt is slurried in a mixture of aqueous sodium carbonate and ethyl acetate and the two phases are then separated.

Following neutralization, the ortho-amino benzyl halide is subjected to catalytic hydrodehalogenation to provide an ortho-methyl aniline. The reaction mixture should include an appropriate hydrogen halide scavenger, which can be selected from a wide variety of organic and inorganic bases. The scavenger neutralizes hydrogen halide as it is formed and solvent soluble scavengers are preferred. Typical scavengers are triethylamine, pyridine, or diisopropylethylamine. Triethylamine is a particularly preferred scavenger.

Preferred hydrogenation catalysts are metal catalysts, such as palladium, platinum, rhodium, nickel, and the like, which most often are available on porous supports. Most preferred are palladium on charcoal catalysts which are commercially available in concentrations of 1 to 10% palladium. The supported catalysts are generally present in amounts less than 10% by weight of the reaction mixture. The temperature broadly may range from about 10°C to about 100°C, typically from about 20°C to about 40°C, and pressures may range from about 50 psig to about 800 psig. Hydrodehalogenation conditions for any given circumstance once again can be readily determined by one skilled in the art.

The hydrodehalogenation reaction should be carried out as rapidly as possible, since the ortho-amine benzyl halide will tend to react with the ortho-methyl aniline product to form intermolecular alkylation products. The final product can be recovered, for example, by adding water to remove precipitated salts, filtering, washing and drying. The final product can be purified further, if desired, by distillation to separate the aniline products from side products (e.g., dimers, polymers, and the like).

Overall yields of up to 80% or more based on the initial ortho-amino benzyl sulfoxides are achieved by this process.

The following examples are included to better illustrate the practice of this invention. These examples are included for illustrative purposes only and are not, in any way, intended to limit the scope of the invention.

Example 1

A solution of 20.0 grams (0.0844 mol) of 2-methylsulfinylmethyl-6-trifluoromethyl aniline in 250 ml of ethylene dichloride in a 500 ml round bottom flask equipped with an efficient stirrer, an HCl inlet tube, distilling head and thermometer was treated at room temperature with gaseous HCl until the initial tacky precipitate gave way to a cloud mixture (3—5 minutes). The mixture was then heated rapidly to 60—63°C while HCl was bubbled through the mixture. After 10 minutes of heating and HCl treatment, 1 ml of $H_2O$ was added. Heating and HCl treatment were continued until the mixture became clear (generally 10—15 minutes after $H_2O$ addition) (often a very small amount of insoluble material will be observed on the sides of the flask at this point). The clear orangish solution was then further heated (the HCl was stopped at this point) and solvent and $H_2O$ were distilled off until no $H_2O$ remained in the reaction flask (typically 50—60 ml of solvent are removed over 15—20 minutes). The clear yellow solution was then cooled with stirring and HCl bubbling through to 0° to 5°C. The precipitated solid was collected by filtration and washed with 50 ml of cold ethylene dichloride then air dried. Yield of off-white 2-chloromethyl-5-trifluoromethyl anilinium hydrochloride was about 95%, but contains a small amount of water. This material had a melting point of about 70°—75°C. Analysis Calculated for $C_8H_8Cl_2F_3N$: C, 39.05; H, 3.28; N, 5.69; Found: C, 41.36, H, 3.43; N, 5.98.

Example 2

Chlorine (76.0 grams, 1.07 mol) was bubbled into a mechanically stirred solution of 221.0 grams (1.00 mol) of 2-methylthiomethyl-6-trifluoromethyl aniline in 2 liters of ethylene dichloride over 60 minutes while maintaining the reaction temperature below 20°C. A white suspension formed initially, became very thick halfway through the addition, and dissolved to give way to a cloudy solution at the end of the addition. The mixture was then heated to 60°C while maintaining a steady stream of HCl bubbling through. Water (25 ml, 1.4 mol) was added and the HCl treatment was maintained at 60°C for 15 minutes at which point a clear solution was obtained. Excess water was removed by azeotropic distillation (780 ml of solvent were removed) and the resulting solution was cooled to 0°C with a steady stream of HCl bubbling through to precipitate the anilinium chloride. Filtration, washing of the solids with 150 ml of cold ethylene dichloride and air-drying gave 228.0 grams (92.7%) of off-white 2-chloromethyl-6-trifluoromethyl anilinium chloride.

### Example 3

In a variation of the procedure of Example 2, chlorine (6 grams, 0.084 mol) was passed into a solution of 17.5 grams (0.0792 mol) of 2-methylthiomethyl-6-trifluoromethyl aniline in 160 ml of ethylene dichloride at 15—21°C over a 15 minute period. Water (2 ml, 0.11 mol) was added and the cloudy solution was heated to 63°C. The mixture was maintained at 63°C and after 30 minutes a clear solution was obtained. Excess water was removed by azeotropic distillation (65 ml of solvent were removed) and the solution was cooled to 0°C with a stream of HCl bubbling through. The resulting solid anilinium salt was isolated by filtration yielding 17.7 grams (90.9%).

### Example 4

A solution of 2-chloromethyl-6-trifluoromethyl aniline (free aniline) was prepared by combining a mixture of 228 grams (0.927 mol) of 2-chloromethyl-6-trifluoromethyl anilinium hydrochloride and one liter of ethyl acetate with one liter of 10% aqueous $Na_2CO_3$. The resulting ethyl acetate solution (combined with a 100 ml backwash of the aqueous layer) was cooled in ice and 107 grams (1.06 mol) of triethylamine were added as an HCl scavenger. This solution was stirred in a two liter autoclave with 7.35 grams (3.2 weight percent) of 5% Pd/C catalyst at 34°C under an initial $H_2$ pressure of 800 psig. After two hours and 35 minutes, $H_2$ uptake was complete. Water was added to dissolve precipitated salts, the mixture was filtered and the ethyl acetate layer was washed with 800 ml of $H_2O$ and dried (MgSO$_4$). The bulk of the solvent was removed *in vacuo* and the residue was distilled through a 20 cm Vigeux column yielding 136.8 grams (84.3% yield) of 2-methyl-6-trifluoromethyl aniline.

### Example 5

In a variation of the procedure of Example 4, a solution of neutralized chloromethyl aniline was prepared in methyl acetate by treating 37.3 grams (0.152 mol) of 2-chloromethyl-6-trifluoromethyl anilinium hydrochloride with 200 ml of methyl acetate and 200 ml of 10% aqueous $Na_2CO_3$. The resulting methyl acetate solution was shaken with 1.15 grams (3.08 weight percent) of 5% Pd/C catalyst and 15.0 grams (0.149 mol) of triethylamine under 50—60 psig of $H_2$ for two hours. The final reaction mixture was filtered, washed with 200 ml of $H_2O$ and dried (MgSO$_4$). Evaporation provided a mixture containing 18.7 grams (70%) of 2-methyl-6-trifluoromethyl aniline. This represented a 70% yield based on starting materials.

Since modifications will be apparent to those skilled in the art, it is intended that this invention be limited only by the scope of the appended claims.

### Claims

1. The process for preparing ortho-methyl anilines which comprises:

a) reacting an ortho-amino benzyl sulfoxide with a nonoxidizing acid halide in an inert solvent to produce a solution of the corresponding ortho-amino benzyl halide and sulfur by-products;

b) adding to the solution containing said ortho-amino benzyl halide and sulfur by-products a hydrogen halide to form and precipitate an anilinium halide;

c) separating said precipitated anilinium halide from said mixture while retaining the sulfur compounds in solution;

d) neutralizing said anilinium salt and forming a solution of ortho-amino benzyl halide in an inert solvent; and

e) catalytically hydrodehalogenating said ortho-amino benzyl halide to form an ortho-methyl aniline.

2. The process of claim 1 wherein said starting sulfoxide is an ortho-methylsulfinylmethyl aniline.

3. The process of claim 1 wherein said starting sulfoxide is a 3-substituted-2-amino benzyl sulfoxide.

4. The process of claim 1 wherein said starting sulfoxide is a 3-trifluoromethyl-2-amino benzyl sulfoxide.

5. The process of claim 1 wherein said starting sulfoxide is 2-methylsulfinylmethyl-6-trifluoromethyl aniline.

6. The process of claim wherein said acid halide is selected from the group consisting of acyl halides, haloacyl halides and inorganic acid halides.

7. The process of claim 6 wherein said acid halide is hydrogen halide.

8. The process of claim 7 wherein said hydrogen halide is hydrogen chloride.

9. The process of claim 1 wherein said hydrogen halide is hydrogen chloride.

10. The process of claim 1 wherein said solvent for step d) is an organic ester.

11. The process of claim 1 wherein a 3-trifluoromethyl-2-amino benzyl sulfoxide is reacted with hydrogen chloride in step a); and hydrogen chloride is also employed in step b).

12. The process of claim 11 wherein an aqueous solution of a base is employed for the neutralization of step d).

13. The process of claim 1 wherein said ortho-amino benzyl sulfoxide is produced by *in situ* reaction of an ortho-amino benzyl sulfide with a halogenating agent to produce an aromatic cyclic sulfilimine intermediate which is hydrolyzed by addition of water to ortho-amino benzyl sulfoxide.

14. The process of claim 1 wherein said ortho-amino benzyl sulfoxide is derived from a ortho-amino benzyl sulfide produced by an aniline sulfilimine rearrangement.

## Patentansprüche

1. Verfahren zur Herstellung von ortho-Methylanilinen, worin

a) ein ortho-Aminobenzylsulfoxid mit einem nichtoxidierenden Säurehalogenid in einem inerten Lösungsmittel umgesetzt wird, um eine Lösung des entsprechenden ortho-Aminobenzylhalogenids und Schwefel-Nebenprodukten herzustellen,

b) der Lösung, welche das ortho-Aminobenzylhalogenid und die Schwefel-Nebenprodukte enthält, ein Halogenwasserstoff zugesetzt wird, um eine Aniliniumhalogenid zu bilden und auszufällen,

c) das ausgefällte Aniliniumhalogenid aus dem Gemisch abgetrennt wird, während die Schwefelverbindungen in Lösung bleiben,

d) das Aniliniumsalz neutralisiert wird und eine Lösung von ortho-Aminobenzylhalogenid in einem inerten Lösungsmittel gebildet wird, und

e) das ortho-Aminobenzylhalogenid katalytisch hydrodehalogeniert wird, um ein ortho-Methylanilin zu bilden.

2. Verfahren nach Anspruch 1, worin das Ausgangs-Sulfoxid ein ortho-Methylsulfinylmethylanilin ist.

3. Verfahren nach Anspruch 1, worin das Ausgangs-Sulfoxid ein 3-substituiertes-2-Aminobenzylsulfoxid ist.

4. Verfahren nach Anspruch 1, worin das Ausgangs-Sulfoxid ein 3-Trifluormethyl-2-aminobenzylsulfoxid ist.

5. Verfahren nach Anspruch 1, worin das Ausgangs-Sulfoxid 2-Methylsulfinylmethyl-6-trifluormethylanilin ist.

6. Verfahren nach Anspruch 1, worin das Säurehalogenid unter Acylhalogeniden, Halogenacylhalogeniden und anorganischen Säurehalogeniden ausgewählt ist.

7. Verfahren nach Anspruch 6, worin das Säurehalogenid Halogenwasserstoff ist.

8. Verfahren nach Anspruch 7, worin der Halogenwasserstoff Chlorwasserstoff ist.

9. Verfahren nach Anspruch 1, worin der Halogenwasserstoff Chlorwasserstoff ist.

10. Verfahren nach Anspruch 1, worin das Lösungsmittel für Stufe d) ein organischer Ester ist.

11. Verfahren nach Anspruch 1, worin ein 3-Trifluoromethyl - 2 - aminobenzylsulfoxid mit Chlorwasserstoff in Stufe a) umgesetzt wird und. Chlorwasserstoff ebenfalls in Stufe b) eingesetzt wird.

12. Verfahren nach Anspruch 11, worin eine wäßrige Lösung einer Base für die Neutralisation in Stufe d) eingesetzt wird.

13. Verfahren nach Anspruch 1, worin das ortho-Aminobenzylsulfoxid durch eine in situ-Reaktion eines ortho-Aminobenzylsulfids mit einem Halogenierungsmittel hergestellt wird, um eine aromatische cyclische Sulfilimin-Zwischenverbindung herzustellen, die durch Zugabe von Wasser zum ortho-Aminobenzylsulfoxid hydrolysiert wird.

14. Verfahren nach Anspruch 1, worin das ortho-Aminobenzylsulfoxid aus einem ortho-Aminobenzylsulfid abgeleitet ist, welches durch eine Anilin-Sulfilimin-Umlagerung hergestellt wird.

## Revendications

1. Procédé de préparation d'ortho-méthyl anilines qui consiste:

a) à faireréagir un sulfoxyde d'ortho-amino benzyle avec un halogénure d'acide non oxydant dans un solvant inerte pour produire une solution de l'halogénure d'ortho-amino benzyle correspondant et des sous-produits soufrés;

b) à ajouter à la solution contenant cet halogénure d'ortho-amino benzyle et ces sous-produits soufrés un halogénure d'hydrogène pour former et précipiter un halogénure d'anilinium;

c) à séparer cet halogénure d'anilinium précipité de ce mélange tout en conservant les composés soufrés en solution;

d) à neutraliser ce sel d'anilinium et à former une solution d'halogénure d'ortho-amino benzyle dans un solvant inerte; et

e) à hydrodéshalogéner catalytiquement cet halogénure d'ortho-amino benzyle pour former une ortho-méthyl aniline.

2. Procédé selon la revendication 1, caractérisé en ce que ce sulfoxyde de départ est une ortho-méthylsulfinylméthyl aniline.

3. Procédé selon la revendication 1, caractérisé en ce que ce sulfoxyde de départ est un sulfoxyde de 2-amino benzyle 3-substitué.

4. Procédé selon la revendication 1, caractérisé en ce que ce sulfoxyde de départ est un sulfoxyde de 3 - trifluorométhyl - 2 - amino benzyle.

5. Procédé selon la revendication 1, caractérisé en ce que ce sulfoxyde de départ est une 2-méthylsulfinylméthyl-6-trifluorométhyl aniline.

6. Procédé selon la revendication 1, caractérisé en ce que cet halogénure d'acide est choisi dans le groupe constitué des halogénures d'acyle, des halogénures d'haloacyle et des halogénures d'acides minéraux.

7. Procédé selon la revendication 6, caractérisé en ce que cet halogénure d'acide est un halogénure d'hydrogène.

8. Procédé selon la revendication 7, caractérisé en ce que cet halogénure d'hydrogène est du chlorure d'hydrogène.

9. Procédé selon la revendication 1, caractérisé en ce que cet halogénure d'hydrogène est du chlorure d'hydrogène.

10. Procédé selon la revendication 1, caractérisé en ce que ce solvant pour le stade d) est un ester organique.

11. Procédé selon la revendication 1, caractérisé en ce qu'on fait réagir un sulfoxyde de 3-trifluorométhyl-2-amino benzyle avec du chlorure d'hydrogène dans le stade a) et en ce qu'on utilise également du chlorure d'hydrogène dans le stade b).

12. Procédé selon la revendication 11, caractérisé en ce qu'on utilise une solution aqueuse d'une base pour la neutralisation du stade d).

13. Procédé selon la revendication 1, caractérisé en ce que ce sulfoxyde d'ortho-amino benzyle est produit par une réaction in situ d'un sulfure d'ortho-amino benzyle avec un agent d'halogénation pour produire une sulfilimine cyclique aromatique intermédiaire qui est hydrolysée par addition d'eau au sulfoxyde d'ortho-amino benzyle.

14. Procédé selon la revendication 1, caractérisé en ce que ce sulfoxyde d'ortho-amino benzyle provient d'un sulfure d'ortho-amino benzyle produit par un réarrangement de sulfilimine de l'aniline.